# EUROPEAN PATENT SPECIFICATION

(11) **EP 3 612 241 B1**
(45) Date of publication and mention of the grant of the patent: **09.06.2021**
(21) Application number: 18719815.5
(22) Date of filing: 18.04.2018
(51) Int. Cl.: A61M 1/00, A61F 13/00, A61F 13/02, A61M 39/24

(54) **WOUND CARE SYSTEM FOR REDUCED PRESSURE THERAPY**
WUNDPFLEGESYSTEM FÜR DRUCKREDUZIERTEN THERAPIE
SYSTÈME DE SOIN DES PLAIES TRAITEMENT À PRESSION RÉDUITE

(30) Priority: 20.04.2017 DE 102017003826
(43) Date of publication of application: 26.02.2020
(73) Proprietor: Lohmann & Rauscher GmbH, 2525 Schönau/Triesting (AT)
(72) Inventor: HENTRICH, Axel, 1060 Wien (AT); SIX-SUDERA, Edda, 1230 Wien (AT); BRÜGGEMANN, Heinrich-Maria, 89597 Munderkingen (DE); SCHRÖDER-DUBOIS, Ludwig, 99084 Erfurt (DE)
(74) Representative: Seranski, Klaus
(86) International application number: PCT/EP2018/059913
(87) International publication number: WO 2018/192978

(56) References cited:
- WO-A1-2015/123340
- FR-A- 1 163 907
- US-A1- 2010 137 775
- US-A1- 2012 238 971
- US-A1- 2012 316 538

## Description

The invention relates to a wound care system as specified in the appended claim 1.

It has been shown that not only the healing of chronic wounds, but also the healing of acute wounds can be assisted by applying reduced pressure to these wounds. Corresponding wound care systems are specified, for example, in EP-A-2822613. With the wound care systems described in this specification the wound space is filled with an absorbent filling material. A gas-tight but water vapour permeable covering device in the form of a covering film, which may comprise, for example, a polyurethane polymer, in particular an aromatic polyurethane polymer, is attached to the side of the filling material facing away from the base of the wound, and is fastened, fluid-tight, to the skin surrounding the wound. For this purpose the boundary surface of the covering film facing the skin can be provided, at least in some areas, with at least one adhesive agent.

In the known wound care system a suction port is attached to the side of the covering device facing away from the filling material, by means of which the wound space can be connected to a pump designed to generate reduced pressure. In wound care systems of this type there is disposed between the suction port and the pump a tube which allows the patient to have a certain degree of mobility during the reduced pressure therapy. Further wound care systems for reduced pressure therapy are disclosed in US 2012/316538 A1, WO 2015/123340 A1, FR 1 163 907 A, US 2012/238971 A1 and US 2010/137775 A1.

The aim of current developments in the field of reduced pressure therapy is to provide portable systems in which the pumps are designed to be smaller and lighter. This applies in particular to the treatment of slightly to moderately exuding wounds. For the treatment of these wounds within the framework of reduced pressure therapy, purely mechanically operated pumps can be used which are connected by a tube to a modern wound dressing. This wound dressing may comprise a covering device and a wound filling material. In order to avoid the use of an exudate canister, which is generally otherwise required within the framework of reduced pressure therapy, an absorbent material, such as for example a superabsorber, may be used in the region of the filling material. The aim of the development of corresponding systems is to avoid burdening the patient when carrying the latter and to restrict his or her freedom of movement as little as possible. However, it has been shown that within the framework of the use of corresponding wound care systems there have in many cases only been poor therapy results.

In view of these problems in the prior art, the object underlying the invention is to provide a wound care system of the type specified at the start which improves the mobility of a patient and with which the desired therapy result can be achieved with improved reliability.

According to the invention this object is achieved by a further development of the known wound care systems which is essentially characterised in that a valve system is assigned to the covering device, said valve system being operable on the one hand to suck gases out of the wound space and on the other hand to counter the penetration of gases into the wound space after fixing the covering device to the skin surrounding the wound, the valve system being able to be coupled detachably to a suction device that can be operated to suck gases out of the wound space.

This invention is based on the finding that the failure of the therapy observed with the conventional wound care systems is in many cases explained by the fact that unnoticed leaks occur in the region of the connection tubes between the suction port of the wound covering and the pump. Due to these leakages, gas penetrates into the wound space and the pressure in the wound space is increased. Then reduced pressure therapy no longer takes place.

Within the framework of this invention it has been discovered that these deficiencies can be eliminated by using pumps with a modular structure. A pump designed to generate reduced pressure generally comprises at least two valves, one of which enables the discharge of gases from the volume to be evacuated, in this case therefore the wound space, but counters the entry of gases into the volume to be evacuated, whereas the other valve enables the discharge of gases sucked out of the volume to be evacuated into the surrounding area.

In wound care systems according to the invention only one of these valve systems which is realized by a film valve is assigned directly to the covering device or to the wound space, whereas the other valve system can, if so required, be coupled detachably thereto in order to generate the desired reduced pressure in the wound space. After the corresponding suction procedure the suction device can be detached from the valve system, which subsequently counters the entry of ambient air into the wound space and so maintains the reduced pressure in the wound space.

The invention has been explained above in connection with the use of the valve system assigned to the covering device as a component part of a pump that can be used to produce reduced pressure. However, the invention can also be used in combination with conventional pumps, the suction port of which is coupled to the valve system assigned to the covering device.

With wound care systems according to the invention the reduced pressure can be maintained over a period of a number of hours despite the uptake of exudate, possibly with the use of a wound material containing an absorbent material, such as for example a superabsorber. The suction device itself does not necessarily have to be carried along over this period of time. In any case, it is not necessary to couple the suction device itself to the covering device with the aid of troublesome tubes. If so required, it can be coupled to the valve device assigned to the covering device. Small and light suction devices, possibly in the form of small mechanical or electromechanical pumps which the patient may, if so required, carry with him or her, may be used here. He/she may, for example, carry them discretely in garment pockets.

Within the framework of the invention it has proven to be particularly advantageous if the valve system has a valve body with a valve chamber serving to receive gases from the wound space. Preferred embodiments of the invention are specified in the dependent claims.

The invention is explained below with reference to the drawings to which reference is explicitly made with regard to all of the details which are essential to the invention and are not elaborated any further in the description. The drawings show as follows:
- Fig. 1: a wound care system according to the invention after application to a wound,
- Fig. 2: a valve system of a wound care system according to the invention according to a first embodiment of the invention,
- Fig. 3: a valve system of a wound care system according to the invention according to a second embodiment of the invention,
- Fig. 4: a valve system of a wound care system according to the invention according to a third embodiment of the invention,
- Fig. 5: an indicator device of a wound care system according to the invention according to a fourth embodiment of the invention,
- Fig. 6: an indicator device of a wound care system according to the invention according to a fifth embodiment of the invention,
- Fig. 7: a suction device of a wound care system according to the invention in a first operating position,
- Fig. 8: the suction device according to Fig. 4 in a second operating position,
- Fig. 9: a combined valve and indicator device of a wound care system according to the invention according to a sixth embodiment of the invention, and
- Fig. 10: a schematic illustration intended to explain the function of the sixth embodiment of the invention,
- Fig. 11: a sectional illustration of a seventh embodiment of the invention,
- Fig. 12: a sectional illustration of an eight embodiment of the invention, and
- Fig. 13: a suction device that can be used in association with the embodiment of the invention illustrated in Fig. 12.

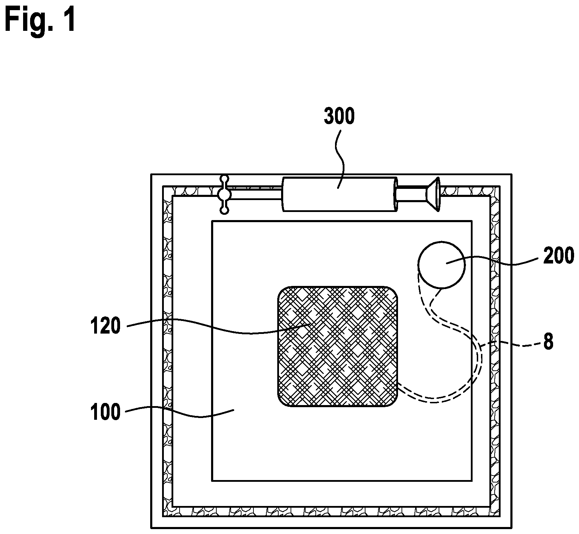

The wound care system illustrated in Fig. 1 essentially comprises a covering device 100 realised in the form of a transparent, gas-tight and preferably water vapour permeable polyurethane film, a wound filling material 120 and a valve system identified as a whole by 200 which is connected to the wound space by means of a tube 8. The polyurethane film 100 is provided on its side facing the skin with an adhesive agent. The tube 8 runs between the covering device 100 and the skin and leads into the wound space configured with the wound filling material 120. Assigned to the valve system 200 is a suction device 300 that can be detachably coupled to the latter in the form of a piston pump which, in the operating position illustrated in Fig. 1, is detached from the valve system 200. In the wound space configured with the wound filling material 120, reduced pressure can be generated by the tube 8 and the valve system 200 with the aid of the suction device 300.

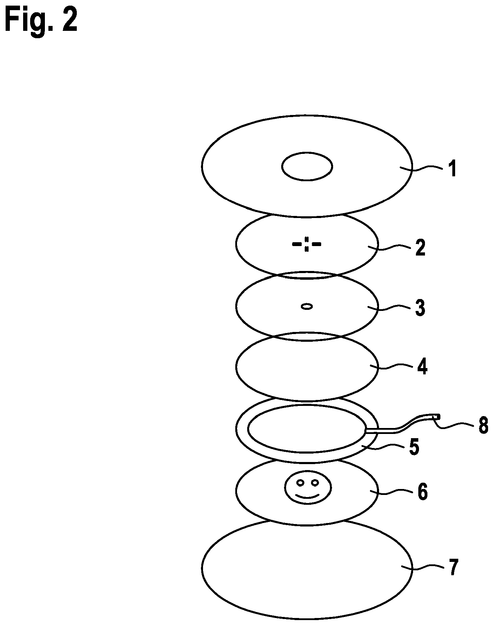

In Fig. 2 a valve system with an integrated indicator device of a wound care system according to a first embodiment of the invention is illustrated in exploded form. The valve system comprises two limiting elements 2 and 7 which are connected to one another in the region of their edges, optionally indirectly, forming a seal, so as to form a valve chamber. The valve chamber that is formed in this way is in the form of a lens and serves to receive gases from the wound space via the tube 8. In the limiting element 2 valve slots are provided to form a one-way valve or check valve. The one-way valve makes it possible to discharge gases from the valve chamber and counters the conveyance of gases from the surrounding area into the valve chamber. Accordingly, gases can only be conveyed into the valve chamber via the tube 8, and so out of the wound space. The limiting element 2 is covered with a reinforcement strip 1, the reinforcement strip 1 having a recess in order to keep the valve slots free. Disposed between the limiting elements 2 and 7 is a film 3 additionally provided with a hole. An indicator device is held between the film 3 and the limiting element 7. The indicator device comprises two indicator layers 4 and 6 running spaced apart and parallel to one another as well as a spacer layer 5 made of compressible material disposed between the indicator layers 4 and 6. The spacer layer 5 is made to be annular in form and the tube 8 passes through it. An indicator space is formed between the indicator layers 4 and 6 and the spacer layer 5.

Due to a pressure difference between the pressure in the indicator space, which because of the connection via the tube 8 corresponds to the pressure in the wound space, and the ambient pressure, the indicator layer 4 is pushed against the pre-tensioning force brought about by the spacer layer 5 against the indicator layer 6. The spacer layer 5 bringing about the pre-tensioning force can have different types of elastic elements with a defined restoring force, such as for example springs, e.g. helical or disc springs. However, foam materials or spacer fabrics may also be used which can be compressed against a pre-defined pre-tensioning force. On the boundary surface of the indicator layer 6 facing the indicator layer 4 an image motif is provided which is visible when the indicator layer 4 comes into contact with the indicator layer 6. It can thus be monitored whether the reduced pressure in the wound space, which corresponds to the pressure in the indicator space, still meets the requirements.

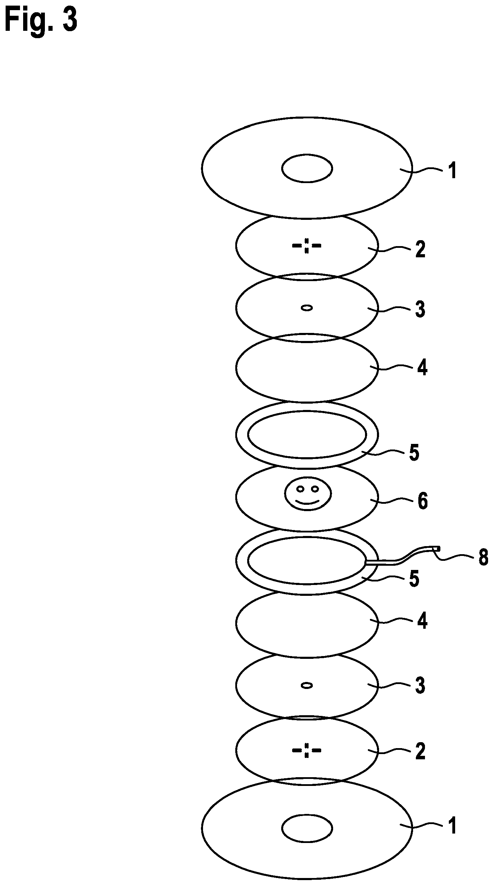

The embodiment of the invention illustrated in Fig. 3 differs substantially from the embodiment of the invention explained by means of Fig. 2 in that an identical structure with a spacer layer 5, an indicator layer 4, a film with an air hole 3 and a limiting element 2 made with valve slots is provided on both sides of the indicator layer 6, in the region of both limiting elements reinforcement layers 1 being provided which have a recess enabling air to escape from the valve chamber formed between the limiting elements 2.

The valve systems of the embodiments of the invention illustrated in Figures 2 and 3 may also comprise film valves in the sense explained above in the region of the limiting elements 2 and 7. In the embodiments of the invention illustrated in Figures 2 and 3 a combination of a film valve and an indicator device is respectively illustrated. In the embodiment illustrated in Fig. 3, there is a film valve on both sides. The valve chamber can then be evacuated from both sides.

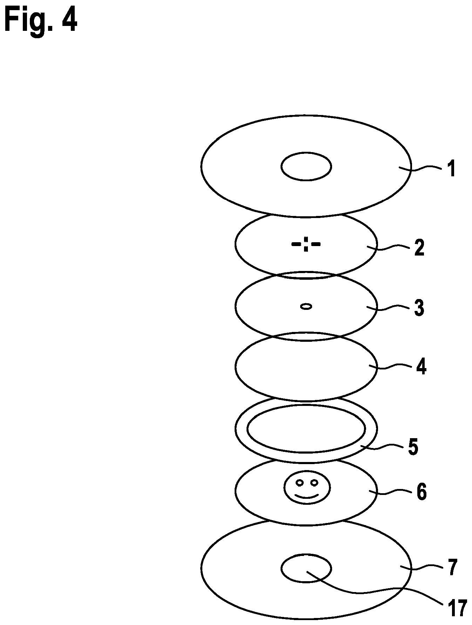

The valve system of a wound care system according to the invention illustrated in Fig. 4 corresponds substantially to the valve system explained by means of Fig. 1. However, the valve system illustrated in Fig. 4 is designed to be applied directly to the covering device of the wound care system. It does not have to be connected to the wound space by means of a fluid line. In the embodiment of the invention illustrated in Fig. 4 the fluid conveying connection to the wound space can be established by means of a hole 17 in the limiting element 7, this limiting element 7 being made in the form of a self-adhesive covering film. This self-adhesive covering film 7' with the centrally positioned hole 17 can be located over a perforation in the covering device (not illustrated). The wound space can be evacuated directly by means of the film valve and the hole 17 in the self-adhesive covering film and the perforation in the covering device. Moreover, the function of the valve system according to Fig. 4 corresponds to the function of the valve system explained by means of Fig. 2.

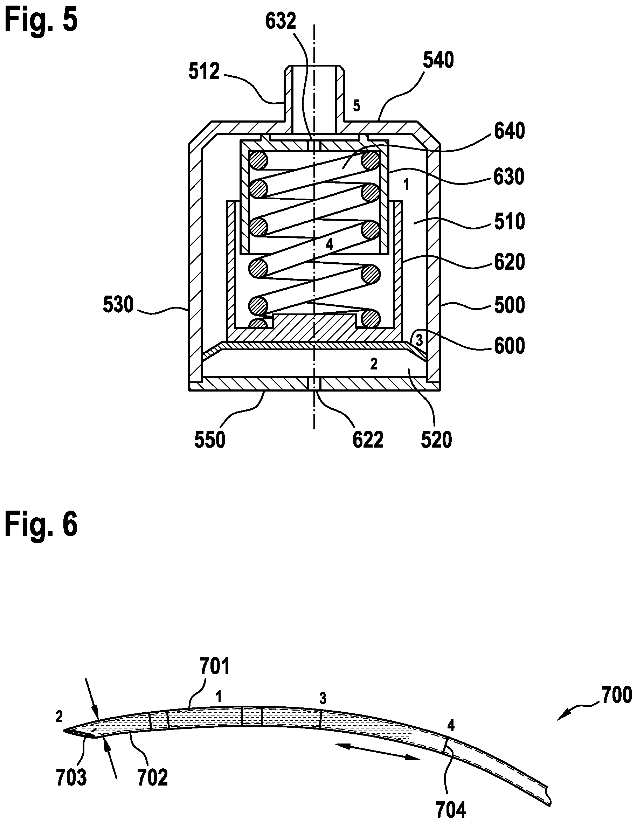

In Fig. 5 an indicator device that can be used within the framework of the invention and that has a rigid housing identified as a whole by 500 is illustrated. The rigid housing 500 is made overall in the form of a circular cylindrical jacket and has two substantially level front surfaces 540 and 550 as well as a jacket 530. The front walls 540, 550 and the jacket 530 of the housing are produced from a transparent material, in particular from a transparent plastic. In the housing 500 two chambers 510 and 520 separated from one another in a gas-tight manner by a deformable membrane 600 are formed. The chamber 520 is connected to the surrounding area by a hole 622 in the front wall 550 of the housing 500. The chamber 510 can be connected fluidically to a wound space by means of a tube port 512 which is disposed in the region of the front surface 540. Two sleeves 620 and 630 which engage in one another and can be displaced with respect to one another are accommodated within the chamber 510. The sleeve 620 is coupled to the membrane 600, whereas the sleeve 630 is coupled to the front wall 540. The sleeves are pushed apart from one another with the aid of a pre-tensioning device 640 made in the form of a compression spring. The jacket of the sleeve 620 coupled to the membrane 600 has a larger diameter than the jacket of the sleeve 630 coupled to the front surface 540, which sleeve 630 is connected to the chamber 610 by means of a hole 632 formed on a face surface thereof. The membrane 600 lies against the front wall 550 of the chamber 520 without generating reduced pressure in the chamber 510. In this initial position the jacket of the sleeve 630 is only covered by the sleeve 620 within the framework of an edge opposite the front wall that has the hole.

If the sleeves 620 and 630 are coloured differently, for example by the sleeve 630 being coloured red and the sleeve 620 being coloured green, it can be seen through the transparent jacket 530 of the housing 500 that there is insufficient reduced pressure in the chamber 510 and so also in the wound space. If reduced pressure is generated in the chamber 510, for example by means of the suction port 512, the membrane 600 may be lifted from the front wall 550 of the chamber 520 due to the corresponding suction effect, pressure equalisation being able to take place in the chamber 520 by means of the opening 622 in this front wall. By lifting the membrane 600 from the front wall 550, the sleeve 620 accommodated in the chamber is pushed towards the front wall 540 of the housing 500 against the pre-tensioning force of the compression spring 640 and so overlaps the sleeve 630. By means of the different colourings of the sleeves 620 and 630, it can thus be signalled through the transparent housing 500 that there is sufficient reduced pressure in the chamber 510, and so also in the wound space.

When the reduced pressure drops, the compression spring 640 pushes the sleeve 620 back in the direction of the front wall 550. The jacket of the sleeve 630 can be seen once again. It is thus signalled through the transparent jacket 530 of the housing 500 that action is necessary in order to obtain the desired reduced pressure status. The concept explained by means of Fig. 5 is also possible in other variations. For example, the two sleeves 620 and 630 can swap positions. For example, the seal between the chambers 510 and 520 can be configured as a flexible setback lip seal.

By applying reduced pressure to the chamber 520 with the aid of an appropriate suction device, air flows through the lip seal from the direction of the chamber 510 and the dressing connected thereto (not illustrated). After completion of the active evacuation the position of the piston (with seal) shifts, according to the equilibrium, from the force resulting from the reduced pressure and the force of the compression spring countering the latter. As a result, the positional relationship of the sleeves 620 and 630 also shifts. The system can be set up so that with the desired reduced pressure status, the sleeve 630 is completely covered by the sleeve 620, and so is no longer visible.

In the indicator device illustrated in Fig. 6, a tube identified as a whole by 700 is used for the visual representation of the reduced pressure status. The tube 700 comprises a reduced pressure-stable tube section 701 and a compressible tube section 702. The two tube sections 701 and 702 are connected to one another in an air- and liquid-tight manner. The tube section 702 is closed, airtight, on its end facing away from the tube section 701. In this way a type of closed blister is produced. The end of the tube section 701 facing away from the tube section 702 is connected fluidically to the wound space. A check valve can also be disposed here between the wound space and the tube section 702.

The tip of the tube 700 which is produced by closing the end of the tube section 702 facing away from the tube section 701 is filled up to a pre-specified mark with a liquid 703. Medical white oil, for example, can be used here. Preferably, the tube as a whole is produced from a polymer which only has a small degree of wettability due to the oil so as to enable a rapid outflow of the liquid if there is a decrease in reduced pressure. The tube section 702 collapses due to the reduced pressure that is generated upon evacuating the wound space. The liquid 703 is pushed out of this region and flows to a second mark 704 on the pressure stable tube section 701. The position of this mark corresponds to the desired reduced pressure level. The reduced pressure status can thus be qualitatively displayed and monitored. In order to avoid any negative impact upon the functional capability of the indicator device illustrated in Fig. 5, for example due to mechanical forces, the entire tube can be encapsulated, for example, by a protective tube. The display of the desired reduced pressure is set by the inside diameter of the tube, the flexibility of the tube material and by the density of the liquid.

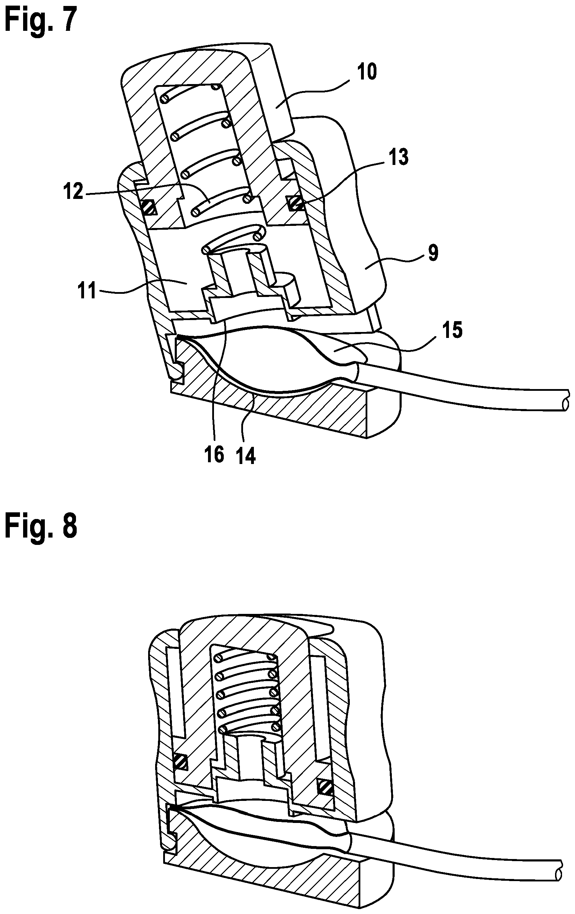

The suction device illustrated in Figures 7 and 8 essentially comprises a housing 9 provided with a sealing flange 16, a piston 10 that can be introduced into the housing against the pre-tensioning force of a spring 12 and a counter-holder 14 that can be moved relative to the housing 9 between an open position, which is illustrated in Fig. 7, and a closed position, which is illustrated in Fig.8. In the open position of the counter-holder 14 illustrated in Fig. 7 a valve body 15 of the type illustrated in Figs. 2 and 3 is introduced into a receiver formed between the housing 9 and the counter-holder 14. A limiting element, provided with valve slots, of the valve body held in the receiver lies against a sealing flange of the housing 9 such that gases can pass out of the valve chamber into the suction chamber formed by the housing 9. By adjusting the counter-holder relative to the housing 9 from the open position illustrated in Fig. 7 into the closed position illustrated in Fig. 8, the valve body 15 is fixed. The fixing of the valve body is brought about so that the sealing flange passes around the one-way valve of the valve body, forming a seal. By actuating the suction device the gases can be sucked out of the valve body and the wound space through the valve slots. In this way the valve body, and so also the wound space, are evacuated. If the suction piston 10 is introduced into the suction chamber 11 against the pre-tensioning force of the compression spring 12, a valve ring lying on a flange of the suction piston is lifted from the flange and makes it possible for gases to escape from the suction chamber 11 into the surrounding area.

If the suction piston 10 is pushed out of the suction chamber 11 by the pre-tensioning force of the spring 12, the valve ring 13 lies against the flange once again and seals off the suction chamber from the surrounding area. The suction that is produced by pushing the suction piston out of the suction chamber by expanding the volume of the suction chamber causes gases from the valve chamber of the valve body 15 to be introduced and so the generation of a reduced pressure in the wound space connected to the valve body 15 by the tube 8. As soon as sufficient reduced pressure has been generated in the wound space, when the counter-holder 14 is open the valve body 15 can be removed from the receiver and the suction device as a whole is mounted independently of the valve system connected to the covering device by the tube 8 and transported. The valve system or the valve body 15 can then be locked in the region of the covering device close to the wound. As soon as a rise in pressure is determined in the valve body 15 or in the wound space with the aid of the indicator device which may be integrated into the valve system, the valve body 15 can once again be coupled to the suction device and gases can once again be sucked out of the wound space.

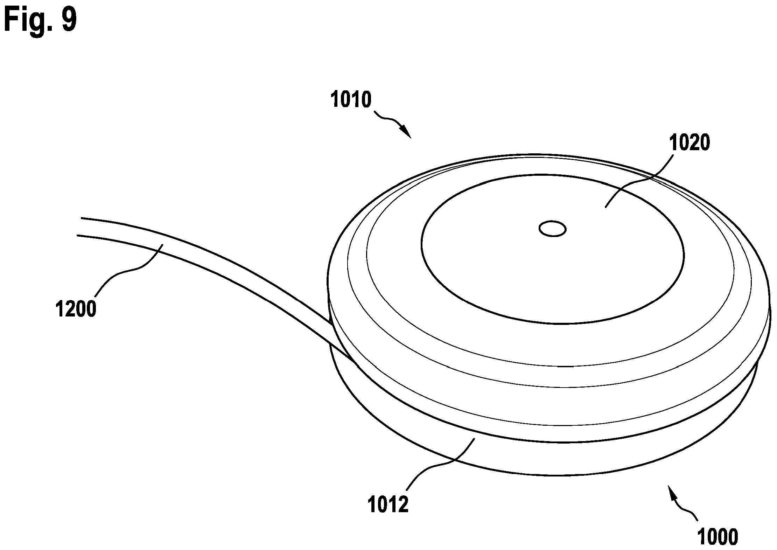

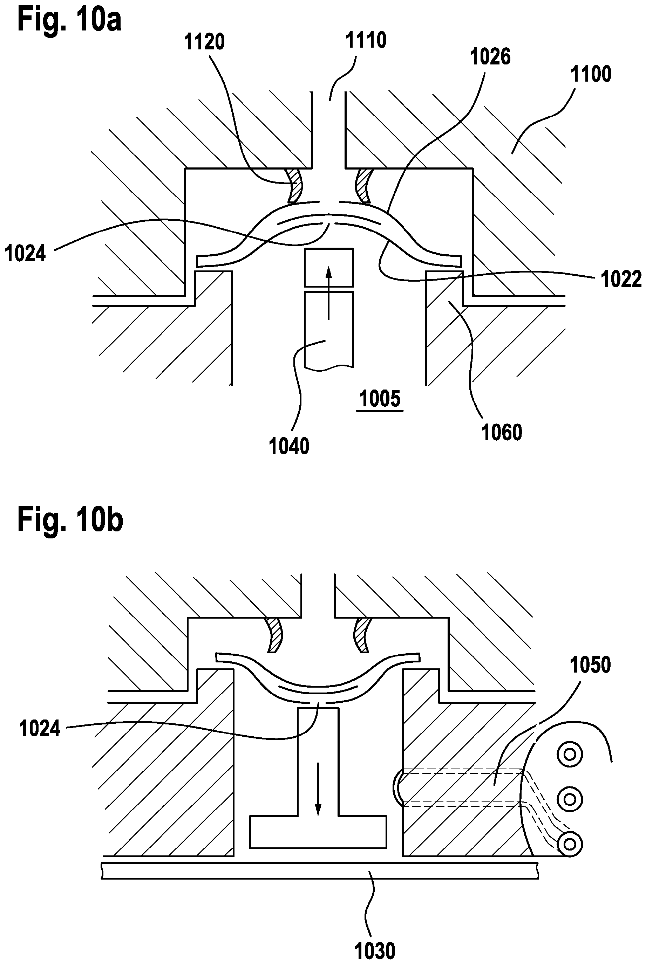

The embodiment of the invention illustrated in Fig. 9 has a combined valve and indicator system identified as a whole by 1000. A chamber 1005 around which a chamber jacket 1010 passes (see Fig. 10) is provided here. The chamber 1005 and the chamber jacket 1010 are made as a whole to be approximately rotationally symmetrical, the height of the substantially cylindrical chamber 1005 being substantially less in the direction of the rotational axis of the chamber jacket 1010 than the diameter of the chamber jacket 1010. A front surface of the chamber 1005 is formed by an indicator wall identified overall by 1020. As can be gathered from the illustration in Fig. 10a and 10b, the indicator wall comprises a snap disc 1022 which curves convexly outwards in the force-free state and through which a recess 1024 passes in the crest region of the curve, and which is fixed to the inner edges of the chamber jacket 1010 adjacent to the chamber 1005.

On its side facing away from the chamber 1005 the recess 1024 is covered by a film valve identified as a whole by 1026. The film valve 1026 makes it possible to suck fluids out of the chamber 1005, but counters the penetration of fluids into the chamber 1005. In this way the chamber 1005 can be evacuated with the aid of a schematically indicated suction device identified as a whole by 1100. On a boundary surface facing the indicator wall in the operating position the suction device 1100 has a seal system 1120 passing around a suction channel 1110 which can be applied to the indicator wall 1020 such that a gas-tight connection between the suction channel 1110 and the indicator wall 1020 is established.

The seal system 1120 passes around a schematically indicated valve slot of the film valve 1026 by means of which a fluid can be sucked out of the chamber 1005 via the suction channel 1110. As soon as reduced pressure is generated in the chamber 1005, there is a pressure difference between the pressure in the chamber 1005 and the pressure outside of the seal system 1120 outside of the seal system 1120. In this way a force pushing the indicator wall 1020 into the chamber 1005 is generated. As soon as the pressure difference is sufficiently great, the snap disc 2011 together with the film valve 1026 disposed on top of it folds down from the initial position illustrated in Fig. 10a into the final position illustrated in Fig. 10b in which the snap disc 1022 is curved into the chamber 1005. The sealing coupling of the suction channel to the indicator wall is released.

The automatic release of the suction channel from the indicator wall is assisted by the chamber jacket having a circumferential collar 1060 made in the form of an annular protrusion which is held in a recess of the suction head of the suction device 1100 so that a front side boundary surface of the suction device 1100 comes into contact with a boundary surface of the chamber jacket 1010 passing around the annular protrusion. The chamber jacket then forms a stop limiting the approach of the seal system 1120 to the snap disc 1022 or the recess 1024 passing through the snap disc so that the seal system 1120 cannot follow the snap disc 1022 into the end position when folded over from the initial position.

It can be determined haptically whether the indicator wall 1020 is curved outwardly (Fig. 10a) or inwardly (Fig. 10b). Thus, the reduced pressure status in the chamber and so also in the wound space connected to the chamber 1005 (see below) can be checked. The snap disc 1022 is made here overall such that in the force-free state, i.e. when the same pressure conditions prevail inside and outside of the chamber 1005, it automatically adopts the initial position illustrated in Fig. 10a. On the side facing away from the indicator wall the chamber 1005 is delimited by an at least partially transparent monitoring wall 1030 on the outer boundary surface of which an adhesive agent, such as for example a hooked strip of a surface hook and loop fastener, can be provided.

Provided within the chamber 1005 is an additional indicator element 1040 which is coupled to the snap disc 1022. If upon reaching a desired reduced pressure status in the chamber 1005 the snap disc 1022 is folded down from the initial position illustrated in Fig. 10a into the final position illustrated in Fig. 10b, a boundary surface of the indicator element 1040 comes into contact with the monitoring wall 1030. As a result, visual monitoring of the reduced pressure status is also made possible in addition to haptic monitoring.

As can be seen in Fig. 10b, a channel 1050 extending in the radial direction passes through the chamber jacket 1010. On its side facing away from the chamber 1005 the channel 1050 leads into a circumferential outer groove 1012 in an outer boundary surface of the chamber jacket 1010. The channel 1050 serves to establish a fluidic connection between the chamber 1005 and the wound space. For this purpose a tube 1200 can be provided which leads into the channel 1050 or is accommodated in the channel 1050 and leads directly into the chamber 1005. The tube 1200 can be wound up in the circumferential groove formed in the chamber jacket. The tube length can thus be set according to the requirements.

The walls of the groove 1012 can be provided with indentations in the region of their ends facing away from the groove bottom. In the region of the indentations the groove width may correspond approximately to the diameter of the tube. Outside of the indentation the groove width may be smaller than the diameter of the tube. Locking of the tube emerging out of the tube at a desired length can thus be brought about. It is then only possible to change the length by slightly squeezing the tube and/or deforming the groove walls, i.e. with additional expenditure of force.

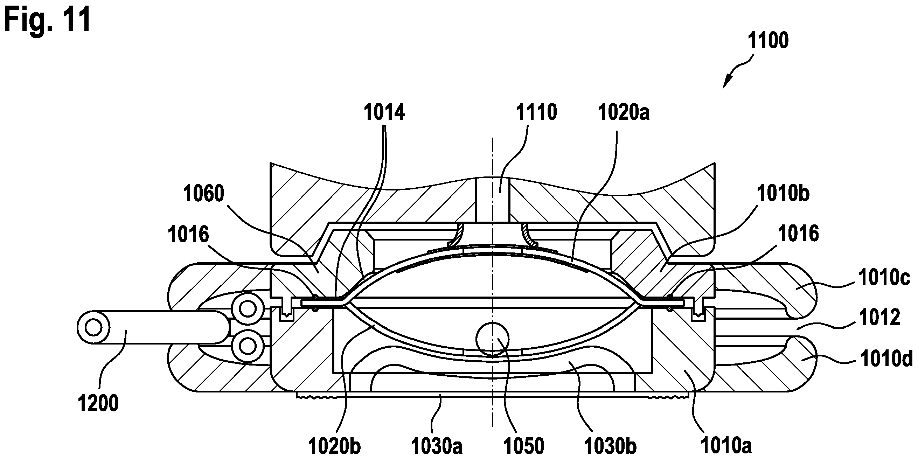

The embodiment of the invention illustrated in Fig. 11 essentially differs from the embodiment explained by means of Fig. 10 in that the chamber jacket 1010 is made in a number of parts. Parts 1010a and 1010b of the chamber jacket 1010 of the embodiment of the invention illustrated in Fig. 11, which lie radially on the inside, are made of a comparably hard plastic material, an inner groove 1014 being formed between the bottom part 1010a and the top part 1010b in which a circumferential edge region of the indicator wall 1020 is accommodated. The indicator wall made in the form of a snap disc is illustrated in the initial position by 1020a and in the final position by 1020b. Sealing rings 1016 are inserted in the region of the walls of the inner groove 1014. In this way the circumferential edge region of the indicator wall 1020 is held, forming a seal, in the inner groove 1014. A space is left free between the bottom of the inner groove 1014 and the edge region of the indicator wall 1020 in the initial position 1020a and the final position 1020b. In this way radial deformation of the indicator wall 1020 when folding down from the initial position into the final position is made possible. The radially outer parts 1010c and 1010d of the chamber jacket 1010 are made of a comparably soft plastic material, and they can be deformed to move the tube 1200 out of the outer groove 1012 formed between the chamber jacket part 1010c and the chamber jacket part 1010d. The axially inwardly pointing edges of the chamber jacket parts 1010c and 1010d may have indentations in the region of which the space between the edges of the chamber jacket parts 1010c and 1010d facing one another corresponds approximately to the tube diameter. In this way a lock can be formed for the tube 1200 emerging out of the outer groove 1012.

In the embodiment of the invention illustrated in Fig. 11, the monitoring wall 1030 is formed from a deformable material. In the final position of the monitoring wall illustrated in Fig. 11 by 1030b, the monitoring wall nestles against the indicator wall 1020b which in this state is in the final position, aided by the pressure difference between the chamber interior and the chamber exterior. In this way visual monitoring of the reduced pressure status in the chamber, and so also in the wound space, can take place. For this purpose, the boundary surface of the indicator wall 1020 facing the monitoring wall 1030 can be provided with profiling or a graphical representation.

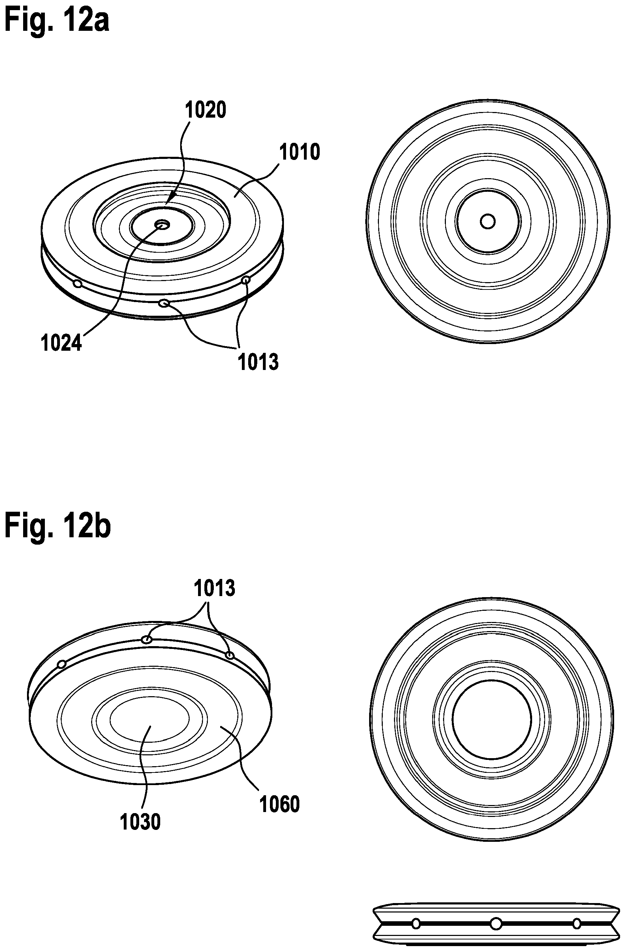

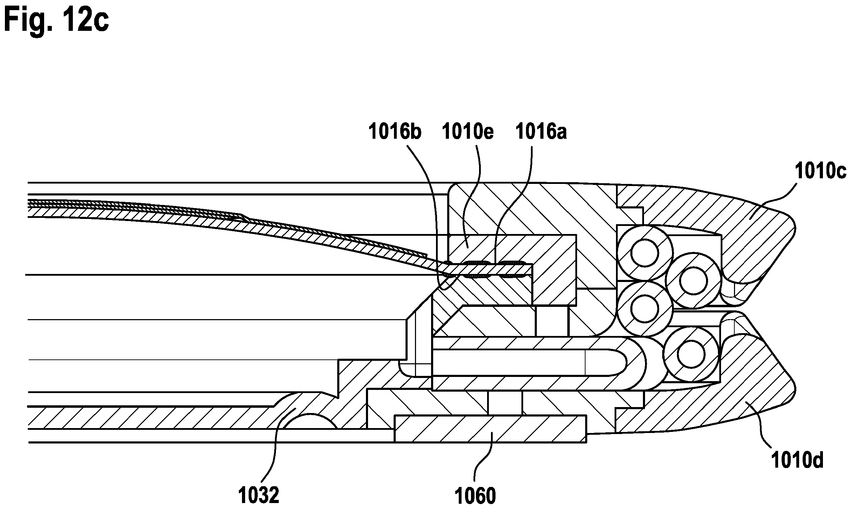

Fig. 12a shows a perspective illustration obliquely from above of an indicator device according to another embodiment of the invention. Fig. 12b shows a perspective illustration obliquely from below of the indicator device according to Fig. 12a, and Fig. 12c shows a sectional illustration of the indicator device according to Figs. 12a and 12b. The embodiment shown in Fig. 12 substantially corresponds in terms of its function to the embodiment illustrated by Fig. 11. It essentially differs from the embodiment illustrated by Fig. 11 in that the cross-over between the indicator wall 1020 and the chamber jacket 1010 is made in the form of a setback so that a receiver for a suction head of a suction device is formed between the chamber jacket 1010 and the indicator wall 1020, the edge of the chamber jacket 1010 lying radially on the inside forming a stop limiting the approach of the suction head to the indicator wall 1020, and so also the recess 1024 passing through the indicator wall.

In Figs. 12b and 12a the indentations in the chamber wall parts 1010c and 1010d, which form a lock for the tube emerging out of the outer groove 1012, can be seen particularly clearly at 1013. As can be seen particularly clearly in Fig. 12b, in the embodiment of the invention illustrated in Fig. 12 a ring 1060 made of adhesive material, preferably in the form of a hooked strip of a surface hook and loop fastener, will pass around the monitoring wall 1030. In this way the attachment of the indicator device to the covering device of a wound care system according to the invention is assisted.

As can be seen in Fig. 12c, the monitoring wall 1030 has a ring 1032 that is curved towards the indicator wall 1020. Due to this annular structure 1032 a sufficiently large amount of material is provided to enable deformation of the monitoring wall 1030, as is required to place the monitoring wall 1030 against the indicator wall in the final position without expanding the material of the monitoring wall. Moreover, the embodiment according to Fig. 12 also differs from the embodiment according to Fig. 11 in that the receiver, forming a seal, of a circumferential edge region of the indicator wall 1020 in the inner groove 1014 is assisted by chamber wall parts 1010e and 1010f made of a flexible material, the chamber wall parts 1010e and 1010f still also having circumferential sealing ribs 1016a projecting into the groove interior.

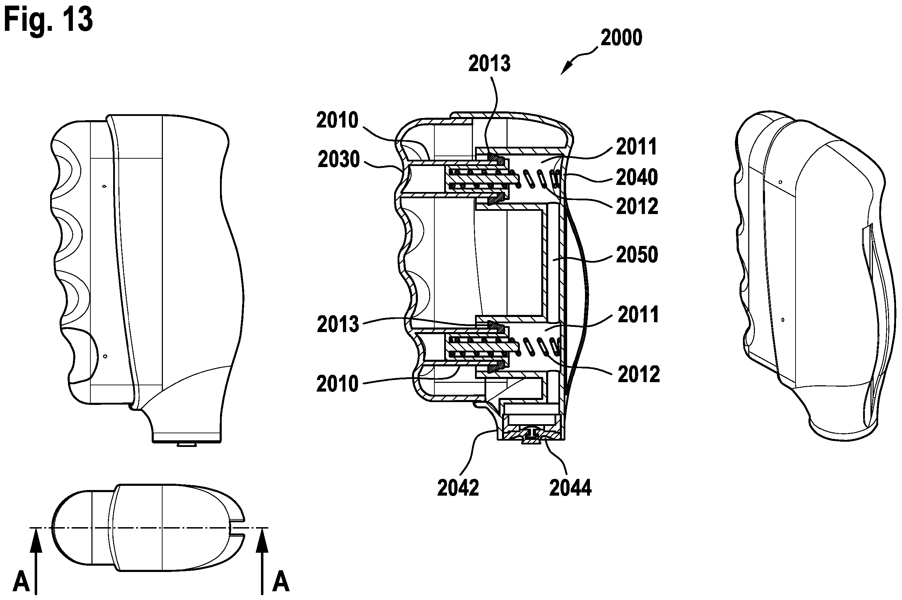

The suction device 2000 illustrated in Fig. 13 and that can be used in association with the indicator device according to Fig. 12 has a housing 2040 and a grip part 2030 that can be pushed out of the initial position illustrated in Fig. 13 into the housing. A suction head 2042, into which a one-way valve 2044 is inserted, is moulded onto the end of the housing 2040 that is shown at the bottom in Fig. 13. The suction head 2042 can be inserted into the receiver of the indicator device around which the chamber jacket 1010 passes. After introducing the suction head 2042 into the receiver, a seal system of the suction head lies against the film valve attached to the outside of the snip disc such that it passes around a recess that passes through the snap disc. In the region of the suction head the one-way valve 2044 facilitates the generation of reduced pressure in the region of the indicator device because reduced pressure can be maintained with the aid of this indicator valve by actuating the suction device between a sealing lip of the suction device 2046, which can be seen particularly clearly in Fig. 11, and the valve system made in the form of a film valve. Due to this reduced pressure, the suction device sucks on the film valve and remains adhered here, even if the suction chambers 2011 of the suction device are ventilated when actuated. In addition or alternatively to the one-way valve 2044 one can also work with a slight leakage flow.

A total of two suction chambers 2011 running parallel to one another are provided in the housing 2040. The grip part 2030 that can be inserted into the housing 2040 has two suction pistons 2010 which can be introduced into the suction chambers 2011. Upon introducing the suction pistons 2010 into the suction chambers 2011, sealing rings 2013 passing around the suction pistons 2010 are lifted from an inner boundary surface of the suction chambers 2011 and thus make it possible for gases to escape from the suction chambers 2011. By introducing the suction pistons 2010 into the suction chambers 2011, compression springs 2012 accommodated in the suction chambers 2011 are compressed. When the suction pistons 2010 are pushed onto the suction chambers 2011 by the effect of the compression springs 2012, the sealing rings 2013 rest against the inner boundary surfaces of the suction chambers 2011. By increasing the volume of the suction chambers 2011, suction is produced in suction channels 2050 which are connected to the suction head 2042. By means of the film valve on the outer boundary surface of the snap disc and the valve system 2044 in the suction head 2042, gases can thus be sucked out of the chamber of the indicator device, and so also out of the wound space, and reduced pressure is established in the chamber and in the wound space.

The invention is not restricted to the exemplary embodiments explained by means of the drawings. For example, the following modifications of the embodiment illustrated by the drawings is also conceivable:
The valve system, possibly in the form of a film valve, and the indicator device are integrated, in combination, directly into the dressing.

The valve system and the indicator device, in combination, as illustrated by the drawings, are connected by a tube to the dressing.

The valve system and the indicator device are connected individually by means of at least one tube to the dressing.

The valve system and the indicator device are incorporated individually directly into the dressing.

The valve system is connected by a tube to the dressing, the indicator device being incorporated directly into the dressing.

The indicator device is connected by a tube to the dressing, the valve system being incorporated directly into the dressing.

The valve system and the indicator device, already pre-fabricated in combination, are applied adhesively to the dressing. For this purpose, the dressing or the covering device is provided at the appropriate point with a recess or is made to be permeable to gas.

The valve system and the indicator device, already individually pre-fabricated, are applied adhesively to the covering device. For this purpose, the covering device is respectively provided at the appropriate points with a recess and a region that is permeable to gas.

With systems according to the invention the following advantages are achieved:
A pump securely connected to the wound care system is not required. In fact, if so required, a suction device can be coupled to the valve system from the outside. After reaching the desired reduced pressure in the wound space the suction device can be removed.

Clearly improved comfort results for the user if the otherwise required connection tube to the pump can either be totally dispensed with or, instead of this connection tube or the fluid line, a clearly shortened tube to the valve system can be used.

The patient's mobility is not, as with other systems, additionally restricted by this tube. Even with a tube-bound valve system of the covering device, in comparison with a conventional system provided with a pump, the overall system is much lighter and smaller. The risk of detachment due to inadvertent tearing on the connection tube between the covering device and the pump and associated leakage of the adhesive covering device is thus considerably reduced.

The system with the valve system and the indicator device, in combination, but also separated, is flexible due to its structure. It can be bent and buckled, and so can be worn discretely on the patient and under clothing. The patient can be informed discretely at all times of the reduced pressure status by means of the indicator device and, if need be, re-pump with the aid of a separate suction device.

The system does not require any additional energy sources such as voltage sources (e.g. battery/socket). In this way allowance is made for the defect that single-use devices with electrical voltage sources may lead to constraints relating to operability and sustainability for the users.

The length of a tube or some other connection can be made variable by using an appropriate connection element. The lack of flexibility, which restricts and possibly discourages the mobile users named as the target group, can be reduced. Even after detaching the suction device the valve system is sealed from the outside with respect to water, dust and air.

The suction device can be designed, for example, as an open piston or bellows system. Therefore, in addition to a favourable production and/or purchase price, it is insensitive to water, dust, dirt, etc. However, it can also be realised in the form of an electromechanical, mechanical, hydraulic and/or pneumatic pump.

The design of a combination of a valve system and an indicator device, as illustrated, for example, in Fig. 3, makes it possible to check the reduced pressure status on both sides, and so without any risk of confusion and, if so required, to re-pump.

By using phosphorescent (luminescent) paints or elements when producing valve systems and/or indicator devices, the reduced pressure status can also be made to be visible in the dark.

The wound care system may be designed as a "single-use product" or the suction device may be designed as a "single-patient-use product". Thus, in terms of sustainability, the amounts of consumable materials can be reduced to the greatest possible extent.

### List of reference numbers

- 1: reinforcement strip
- 2: limiting element
- 3: film
- 4: indicator layer
- 5: spacer layer
- 6: indicator layer
- 7: limiting element
- 8: tube
- 9: housing
- 10: piston
- 12: spring
- 13: sealing ring
- 14: counter-holder
- 15: valve body
- 16: sealing flange
- 17: hole
- 100: covering device
- 120: wound filling material
- 200: valve system
- 300: suction device
- 500: housing
- 530: jacket
- 600: membrane
- 640: pre-tensioning device
- 700: tube
- 701: reduced pressure-stable tube section
- 702: compressible tube section
- 703: liquid
- 704: mark
- 510, 520: chambers
- 540, 550: front surface
- 620,630: sleeve
- 622, 632: hole in the front wall
- 1000: valve and indicator system
- 1005: chamber
- 1010: chamber jacket
- 1012: outer groove
- 1014: inner groove
- 1016: sealing rings
- 1020: indicator wall
- 1022: snap disc
- 1024: recess
- 1026: film valve
- 1030: monitoring wall
- 1032: upwardly curved ring
- 1040: indicator element
- 1050: channel
- 1060: annular protrusion (collar)
- 1100: suction device
- 1110: suction channel
- 1120: seal system
- 1200: tube
- 2000: suction device
- 2010: suction piston
- 2011: suction chamber
- 2012: compression spring
- 2013: sealing rings
- 2030: grip part
- 2040: housing
- 2042: suction head
- 2044: one-way valve
- 2050: suction channel

## Claims

1. A wound care system for reduced pressure therapy comprising a covering device (100), which is provided, at least in sections, with an adhesive agent designed to fix the covering device (100) to the skin, preferably running around the wound space, that can be fixed to the skin surrounding a wound and serving to produce a closed wound space containing the wound, comprising a valve system (200, 1000) assigned to the covering device, said valve system (200, 1000) being operable on the one hand to suck gases out of the wound space and on the other hand to counter the penetration of gases into the wound space after fixing the covering device (100) to the skin surrounding the wound, the valve system (200, 1000) being able to be coupled detachably to a suction device (300, 1100, 2000) that can be operated to suck gases out of the wound space, wherein the valve system (200, 1000) is coupled to the covering device (100) by means of a fluid line, in particular a tube (8, 1200) **characterised in that** the valve system (200, 1000) has a valve body (15, 1010) with a valve chamber (1005) serving to receive gases from the wound space, wherein the tube (8, 1200) coupled to the covering device (100) leads into the valve chamber (1005), the valve system (200, 1000) comprises a film valve (1026) and the valve chamber (1005) is at least partially delimited by a rigid housing (1010).

2. The wound care system according to Claim 1, **characterised in that** the one-way valve has at least one valve slot formed in a preferably film-like limiting element (2) delimiting the valve chamber.

3. The wound care system according to any of Claims 1 or 2, **characterised in that** the valve chamber is approximately lens-shaped before gases are released from it and is delimited by two film-like limiting elements (2, 7) connected, gas-tight, to one another in the region of their edges, at least one, preferably both of which limiting elements are provided with at least one valve slot in order to form a film valve.

4. The wound care system according to Claim 2 or 3, **characterised in that** at least one limiting element (2) has a reinforcement layer (1) provided with a recess in the region of the at least one valve slot.

5. The wound care system according to any of Claims 1 to 4, **characterised in that** the fluid line (8) is attached to the boundary surface of the covering device provided with the adhesive agent.

6. The wound care system according to any of Claims 1 to 4, **characterised in that** the fluid line (8) passes through the covering device and/or leads into an opening that passes through the sealing device and/or the covering device has a suction port designed for coupling the fluid line.

7. A wound care system according to any of the preceding claims, **characterised in that** an indicator device (4, 700, 1000) that can be operated to display the reduced pressure status in the wound space is assigned to the covering device (100).

8. The wound care system according to Claim 7, **characterised in that** the indicator device is integrated into the covering device (100).

9. The wound care system according to Claim 7, **characterised in that** the indicator device (4, 700, 1000) is coupled by means of a fluid line (8, 1200) to the covering device (100).

10. The wound care system according to any of Claims 7 to 9, **characterised in that** the indicator device (4, 1000) is integrated into the valve system (200, 1000), in particular into the valve chamber (1005) of the valve system (200, 1000).

11. The wound care system according to any of Claims 7 to 10, **characterised in that** the indicator device has two indicator layers (4, 6) spaced apart from one another and running approximately parallel to one another, which can be compressed, at least in some areas, against a pre-tensioning force, so as to reduce the spacing.

12. The wound care system according to Claim 11, **characterised by** at least one in particular elastically deformable spacer layer (5), disposed between the indicator layers (4, 6) and that can be compressed by overcoming a pre-determined pre-tensioning force, preferably having at least one window.

13. The wound care system according to any of Claims 9 to 12, **characterised in that** the fluid line (8) leads into an indicator space formed between the indicator layers (4, 6), and preferably at least partially passes through the spacer layer (5).

14. The wound care system according to any of Claims 11 to 13, **characterised in that** a boundary surface of one indicator layer (6) facing the other indicator layer (4) has profiling and/or graphical representation which can be seen from the outside when the distance between the indicator layers is reduced.

15. The wound care system according to any of Claims 7 to 14, **characterised in that** the indicator device (1000) has an at least partially rigid housing (500, 1010) which at least partially delimits an indicator chamber (510, 1005) serving to receive gases from the wound space.

16. The wound care system according to Claim 15, **characterised in that** two chambers (510, 520) separated from one another by a gas-tight and moveable, in particular deformable, separating device (600) are formed in the indicator chamber, the first chamber (510) of which can preferably be connected to the wound space by means of a fluid line, and the second chamber (520) of which can preferably be connected to the surrounding area by means of a recess (622) in the housing (500).

17. The wound care system according to Claim 16, **characterised by** a pre-tensioning device (540) preferably accommodated in the first chamber (510), in particular comprising a compression spring, by means of which the separating device (600), preferably made in the form of a deformable membrane, is held in an initial position and against the pre-tensioning force of which the separating device (600) is moved to a final position when reduced pressure is applied to the first chamber (510=.

18. The wound care system according to Claim 17, **characterised in that** the separating device (600) comes at least partially into contact with a preferably plane boundary surface of the preferably cylindrical, in particular circular cylindrical, housing (500) upon reaching the end position.

19. The wound care system according to either of Claims 16 or 17, **characterised in that** there are disposed in one of the chambers, preferably in the first chamber (510), two sleeves (620, 630) that engage in one another and that can be displaced relative to one another against the pre-tensioning force of the pre-tensioning device (640), one of which sleeves (620, 630) is coupled to a housing wall (540), and the other of which is coupled to the separating device (600).

20. The wound care system according to any of Claims 16 to 18, **characterised in that** a housing wall delimiting the first chamber of the housing is provided with a one-way valve, in particular a film valve, by means of which reduced pressure can be applied to the first chamber and to the wound space.

21. The wound care system according to any of the preceding claims, **characterised by** a chamber (1005) that is fluidically connected to the wound space, preferably a substantially cylindrical chamber (1005), such as for example the valve chamber and/or the indicator chamber, which is delimited by a circumferential chamber jacket (1010) and has an indicator wall (1020) preferably forming a front side of the chamber (1005) and moveable between an initial position and a final position, the indicator wall (1020) being able to be moved from the initial position into the final position by applying reduced pressure to the chamber (1005).

22. The wound care system according to any of the preceding claims, **characterised in that** in the initial position the indicator wall (1020) is curved convexly outwards towards the space lying outside of the chamber (1005).

23. The wound care system according to Claim 21 or 22, **characterised in that** the indicator wall (1020) is curved into the chamber (1005) in the final position.

24. The wound care system according to any of Claims 21 to 23, **characterised in that** the indicator wall (1020) has an outwardly curved snap disc (1022) in the unstressed state.

25. The wound care system according to Claim 24, **characterised in that** a recess (1024) preferably positioned in the crest region of the curve and serving to apply reduced pressure to the chamber (1005) and the wound space passes through the snap disc (1022).

26. The wound care system according to any of Claims 21 to 25, **characterised in that** a circumferential edge region of the indicator wall (1020) is accommodated in an inner groove (1014) of the chamber jacket (1010) that is open towards the chamber (1005).

27. The wound care system according to Claim 26, **characterised in that** in the initial position and/or the final position of the indicator wall (1020) a space is formed between the bottom of the inner groove and the edge region of the indicator wall (1020).

28. The wound care system according to either of Claims 26 or 27, **characterised in that** regions of the walls of the inner groove coming into contact with the circumferential edge region of the indicator wall (1020) are formed at least partially from a flexible material (1016).

29. The wound care system according to any of Claims 21 to 28, **characterised in that** a circumferential collar (1060) for the form-locking coupling of a suction head of a suction device (1100) is formed in the region of the cross-over between the indicator wall (1020) and the chamber jacket (1010), the collar (1060) preferably forming a stop limiting the approach of the suction head to the recess (1024) passing through the snap disc (1022).

30. The wound care system according to any of Claims 25 to 29, **characterised in that** the indicator wall (1020) has a valve system covering the recess (1024) of the snap disc (1022). preferably in the form of a film valve (1026), serving to suck gases out of the chamber (1005) and countering the penetration of gases into the chamber (1005).

31. The wound care system according to any of Claims 21 to 30, **characterised in that** a channel (1050) leading into the chamber (1005) passes through the chamber jacket (1010).

32. The wound care system according to Claim 27, **characterised in that** a circumferential outer groove (1012) for accommodating a tube (1200) connecting the chamber (1005) to the wound space, and that is preferably wound up in the outer groove (1012), is provided in the boundary surface of the chamber jacket (1010) facing away from the chamber (1005), the channel (1050) leading into the outer groove (1012) on its side facing away from the chamber (1005).

33. The wound care system according to any of Claims 21 to 32, **characterised in that** the groove (1012) has locking means (1013) for locking the tube (1200) emerging out of the groove (1012).

34. The wound care system according to Claim 32 or 33, **characterised in that** a recess passes through at least one wall of the outer groove (1012) in order to discharge liquid from said recess.

35. The wound care system according to any of Claims 21 to 34, **characterised in that** the chamber (1005) is delimited by an at least partially transparent monitoring wall (1030) on the side facing away from the indicator wall (1020).

36. The wound care system according to Claim 35, **characterised in that** an indicator element (1040) coupled to the indicator wall (1020) is disposed in the chamber (1005), which indicator element (1040) is moved towards the monitoring wall (1030) upon reaching the final position and preferably comes into contact with the monitoring wall (1030).

37. The wound care system according to Claim 35 or 36, **characterised in that** the monitoring wall (1030) is deformable and comes into contact with the boundary surface of the indicator wall (1020) facing the chamber (1005) when a pre-determined reduced pressure is applied to the chamber (1005).

38. The wound care system according to any of Claims 7 to 37, **characterised in that** the indicator device has a tube (700) connected fluidically to the wound space, which tube (700) has a reduced pressure-stable section (701) facing the wound space and a compressible section (702) facing away from the wound space and which is closed fluid-tightly on its end facing away from the wound space, the tube (700) being filled partially with a liquid (703), in particular white oil.

39. The wound care system according to any of the preceding claims, **characterised by** a suction device (2000) that can be coupled detachably to the valve system (1000).

40. The wound care system according to Claim 39, **characterised in that** the suction device comprises a sealing flange passing around at least one one-way valve of the valve body (1010, 1020, 1030) and/or of the indicator device in order to establish a sealing connection between the valve chamber (1005) and/or a chamber (1005) of the indicator device (1000) and the suction chamber (2011) enabling gases to pass out of the valve chamber (1005) and/or a chamber of the indicator device into at least one suction chamber (2011) of the suction device (2000), the suction device (2000) preferably having a receiver for the valve body.

41. The wound care system according to Claim 39 or 40, **characterised in that** the suction device (2000) can be operated to suck gases out of the valve chamber of the valve body that is preferably held in the receiver.

42. The wound care system according to any of Claims 39 to 41, **characterised by** at least one suction piston (2010) that can preferably be introduced into the suction chamber (2011) against the pre-tensioning force of a pre-tensioning device 82012).

43. The wound care system according to Claim 42, **characterised in that** at least one further valve system (2013) is assigned to the at least one suction piston (2010), which valve system (2013) allows gases to be released from the suction chamber (2011) to the surrounding area when the suction piston (2010) is introduced into the suction chamber (2011) and counters the penetration of ambient air into the valve chamber (1005) when the suction piston (2010) is removed from the suction chamber (2011).

44. The wound care system according to any of the preceding claims, **characterised by** a wound filling material (120), preferably comprising an absorbent material, such as for example a superabsorber, that can be inserted into the wound space.

45. The wound care system according to any of Claims 39 to 44, **characterised in that** the suction device has a pump, in particular an electromechanical pump.

## Patentansprüche

1. Wundversorgungsanordnung für die Unterdrucktherapie, umfassend eine Abdeckeinrichtung (100), die wenigstens abschnittweise mit einem vorzugsweise um den Wundraum umlaufenden Haftmittel ausgestattet ist, das zum Festlegen der Abdeckvorrichtung (100) an der Haut angepasst ist und zum Herstellen eines die Wunde enthaltenden abgeschlossenen Wundraums an der Haut festgelegt werden kann, umfassend eine der Abdeckeinrichtung zugehörige Ventilanordnung (200, 1000), wobei die Ventilanordnung (200, 1000) nach Festlegen der Abdeckeinrichtung (100) an der die Wunde umgebenden Haut einerseits zum Absaugen von Gasen aus dem Wundraum und andererseits zum Entgegenwirken des Eindringens von Gasen in den Wundraum betreibbar ist, wobei die Ventilanordnung (200, 1000) lösbar an eine zum Absaugen von Gasen aus dem Wundraum betreibbare Saugeinrichtung (300, 1100, 2000) koppelbar ist, wobei die Ventilanordnung (200, 1000) über eine Fluidleitung, insbesondere einen Schlauch (8, 1200), an die Abdeckeinrichtung (100) gekoppelt ist,
**dadurch gekennzeichnet, dass** die Ventilanordnung (200, 1000) einen Ventilkörper (15, 1010) mit einer zum Aufnehmen von Gasen aus dem Wundraum dienenden Ventilkammer (1005) aufweist, wobei der an die Abdeckeinrichtung (100) gekoppelte Schlauch (8, 1200) in die Ventilkammer (1005) mündet, wobei die Ventilanordnung (200, 1000) ein Folienventil (1026) umfasst und die Ventilkammer (1005) zumindest teilweise durch ein starres Gehäuse (1010) begrenzt ist.

2. Wundversorgungsanordnung nach Anspruch 1, **dadurch gekennzeichnet, dass** das Einwegventil mindestens einen in einem die Ventilkammer begrenzenden, vorzugsweise folienartigen Begrenzungselement (2) gebildeten Ventilschlitz aufweist.

3. Wundversorgungsanordnung nach einem der Ansprüche 1 oder 2, **dadurch gekennzeichnet, dass** die Ventilkammer vor dem Absaugen von Gasen daraus etwa linsenförmig ist und von zwei im Bereich ihrer Ränder gasdicht miteinander verbundenen folienartigen Begrenzungselementen (2, 7) begrenzt ist, von denen mindestens eines, vorzugsweise beide, mit mindestens einem Ventilschlitz ausgestattet ist, um ein Folienventil herzustellen.

4. Wundversorgungsanordnung nach Anspruch 2 oder 3, **dadurch gekennzeichnet, dass** mindestens ein Begrenzungselement (2) eine im Bereich des mindestens einen Ventilschlitzes mit einer Aussparung versehende Verstärkungslage (1) aufweist.

5. Wundversorgungsanordnung nach einem der Ansprüche 1 bis 4, **dadurch gekennzeichnet, dass** die Fluidleitung (8) an der mit dem Haftmittel ausgestatteten Begrenzungsfläche der Abdeckeinrichtung angebracht ist.

6. Wundversorgungsanordnung nach einem der Ansprüche 1 bis 4, **dadurch gekennzeichnet, dass** die Fluidleitung (8) die Abdeckeinrichtung durchsetzt und/oder in eine die Abdichteinrichtung durchsetzende Öffnung mündet und/oder die Abdeckeinrichtung einen zum Koppeln der Fluidleitung dienenden Ansauganschluss aufweist.

7. Wundversorgungsanordnung nach einem der vorhergehenden Ansprüche, **dadurch gekennzeichnet, dass** der Abdeckeinrichtung (100) eine zum Darstellen des Unterdruckstatus in dem Wundraum betreibbare Indikatoreinrichtung (4, 700, 1000) zugehörig ist.

8. Wundversorgungsanordnung nach Anspruch 7, **dadurch gekennzeichnet, dass** die Indikatoreinrichtung in die Abdeckeinrichtung (100) integriert ist.

9. Wundversorgungsanordnung nach Anspruch 7, **dadurch gekennzeichnet, dass** die Indikatoreinrichtung (4, 700, 1000) über eine Fluidleitung (8, 1200) an die Abdeckeinrichtung (100) gekoppelt ist.

10. Wundversorgungsanordnung nach einem der Ansprüche 7 bis 9, **dadurch gekennzeichnet, dass** die Indikatoreinrichtung (4, 1000) in die Ventilanordnung (200, 1000) integriert, insbesondere in der Ventilkammer (1005) der Ventilanordnung (200, 1000) angeordnet ist.

11. Wundversorgungsanordnung nach einem der Ansprüche 7 bis 10, **dadurch gekennzeichnet, dass** die Indikatoreinrichtung zwei mit Abstand etwa parallel zueinander verlaufende Indikatorlagen (4, 6) aufweist, die gegen eine Vorspannkraft zumindest bereichsweise unter Verminderung des Abstands zusammengedrückt werden können.

12. Wundversorgungsanordnung nach Anspruch 11, **gekennzeichnet durch** mindestens eine zwischen den Indikatorlagen (4, 6) angeordnete und unter Überwindung einer vorgegebenen Vorspannkraft kompressible, insbesondere elastisch verformbare, vorzugsweise mindestens ein Fenster aufweisende Distanzlage (5).

13. Wundversorgungsanordnung nach einem der Ansprüche 9 bis 12, **dadurch gekennzeichnet, dass** die Fluidleitung (8) in einen zwischen den Indikatorlagen (4, 6) gebildeten Indikatorraum mündet, und vorzugsweise die Distanzlage (5) zumindest teilweise durchsetzt.

14. Wundversorgungsanordnung nach einem der Ansprüche 11 bis 13, **dadurch gekennzeichnet, dass** eine der anderen Indikatorlage (4) zugewandte Begrenzungsfläche einer Indikatorlage (6) eine Profilierung und/oder graphische Darstellung aufweist, die bei Verminderung des Abstands zwischen den Indikatorlagen erkennbar wird.

15. Wundversorgungsanordnung nach einem der Ansprüche 7 bis 14, **dadurch gekennzeichnet, dass** die Indikatoreinrichtung (1000) ein zumindest teilweise starres Gehäuse (500, 1010) aufweist, das zumindest teilweise eine zum Empfangen von Gasen aus dem Wundraum dienenden Indikatorkammer (510, 1005) begrenzt.

16. Wundversorgungsanordnung nach Anspruch 15, **dadurch gekennzeichnet, dass** zwei durch eine gasdichte und bewegliche, insbesondere verformbare Trennvorrichtung (600) voneinander getrennte Kammern (510, 520) in der Indikatorkammer ausgebildet sind, von denen die erste Kammer (510) vorzugsweise mittels einer Fluidleitung mit dem Wundraum verbunden sein kann und die zweite Kammer (520) vorzugsweise mittels einer Aussparung (622) in dem Gehäuse (500) mit der Umgebung verbunden sein kann.

17. Wundversorgungsanordnung nach Anspruch 16, **gekennzeichnet durch** eine vorzugsweise in der ersten Kammer (510) untergebrachte Vorspannvorrichtung (640), die insbesondere eine Druckfeder umfasst, mit deren Hilfe die vorzugsweise in Form einer verformbaren Membran hergestellte Trennvorrichtung (600) in einer Ausgangsposition und gegen deren Vorspannkraft die Trennvorrichtung (600) gehalten wird, in eine Endposition gebracht wird, wenn auf die erste Kammer (510) ein Unterdruck ausgeübt wird.

18. Wundversorgungsanordnung nach Anspruch 17, **dadurch gekennzeichnet, dass** die Trennvorrichtung (600) beim Erreichen der Endposition zumindest teilweise mit einer vorzugsweise ebenen Begrenzungsfläche des vorzugsweise zylindrischen, insbesondere kreisförmigen zylindrischen Gehäuses (500) in Kontakt kommt.

19. Wundversorgungsanordnung nach einem der Ansprüche 16 und 17, **dadurch gekennzeichnet, dass** in einer der Kammern, vorzugsweise in der ersten Kammer (510), zwei Hülsen (620, 630) angeordnet sind, die ineinander greifen und die relativ zueinander gegen die Vorspannkraft der Vorspannvorrichtung (640) verschoben werden können, wobei eine der Hülsen (620, 630) mit einer Gehäusewand (540) und die andere mit der Trennvorrichtung (600) gekoppelt ist.

20. Wundversorgungsanordnung nach einem der Ansprüche 16 bis 18, **dadurch gekennzeichnet, dass** eine die erste Kammer des Gehäuses begrenzende Gehäusewand mit einem Einwegventil, insbesondere einem Folienventil, versehen ist, mit dessen Hilfe ein Unterdruck auf die erste Kammer und den Wundraum ausgeübt werden kann.

21. Wundversorgungsanordnung nach einem der vorhergehenden Ansprüche, **gekennzeichnet durch** eine fluidisch mit dem Wundraum verbundene Kammer (1005), die vorzugsweise eine im wesentlichen zylindrische Kammer (1005) ist, wie beispielsweise die Ventilkammer und/oder die Indikatorkammer, die durch einen umlaufenden Kammermantel (1010) begrenzt ist und eine Indikatorwand (1020) aufweist, die vorzugsweise eine Vorderseite der Kammer (1005) bildet und zwischen einer Anfangsposition und einer Endposition beweglich ist, wobei die Indikatorwand (1020) durch Anlegen eines Unterdrucks an die Kammer (1005) von der Ausgangsposition in die Endposition bewegt werden kann.

22. Wundversorgungsanordnung nach einem der vorhergehenden Ansprüche, **dadurch gekennzeichnet, dass** die Indikatorwand (1020) in der Ausgangsposition konvex nach außen in Richtung des außerhalb der Kammer (1005) liegenden Raums gekrümmt ist.

23. Wundversorgungsanordnung nach Anspruch 21 oder 22, **dadurch gekennzeichnet, dass** die Indikatorwand (1020) in der Endposition in die Kammer (1005) gekrümmt ist.

24. Wundversorgungsanordnung nach einem der Ansprüche 21 oder 23, **dadurch gekennzeichnet, dass** die Indikatorwand (1020) im nicht belasteten Zustand eine nach außen gekrümmte Schnappscheibe (1022) aufweist.

25. Wundversorgungsanordnung nach Anspruch 24, **dadurch gekennzeichnet, dass** eine vorzugsweise im Scheitelbereich der Kurve positionierte Aussparung (1024) dazu dient, einen Unterdruck auf die Kammer (1005) auszuüben und der Wundraum durch die Schnappscheibe (1022) verläuft.

26. Wundversorgungsanordnung nach einem der Ansprüche 21 bis 25, **dadurch gekennzeichnet, dass** ein Umfangskantenbereich der Indikatorwand (1020) in einer inneren Nut (1014) des zur Kammer (1005) hin offenen Kammermantels (1010) untergebracht ist.

27. Wundversorgungsanordnung nach Anspruch 26, **dadurch gekennzeichnet, dass** in der Anfangsposition und/oder der Endposition der Indikatorwand (1020) ein Raum zwischen dem Boden der inneren Nut und dem Randbereich der Indikatorwand (1020) gebildet wird.

28. Wundversorgungsanordnung nach einem der Ansprüche 26 oder 27, **dadurch gekennzeichnet, dass** mit dem Umfangskantenbereich der Indikatorwand (1020) in Kontakt kommende Bereiche der Wände der inneren Nut zumindest teilweise aus einem flexiblen Material (1016) gebildet sind.

29. Wundversorgungsanordnung nach einem der Ansprüche 21 bis 28, **dadurch gekennzeichnet, dass** im Kreuzungsbereich zwischen der Indikatorwand (1020) und dem Kammermantel (1010) ein Umfangskragen (1060) zur Formschlusskupplung eines Saugkopfes einer Saugvorrichtung (1100) ausgebildet ist, wobei der Kragen (1060) vorzugsweise einen Anschlag bildet, der die Annäherung des Saugkopfes an die durch die Schnappscheibe (1022) verlaufende Aussparung (1024) begrenzt.

30. Wundversorgungsanordnung nach einem der Ansprüche 25 bis 29, **dadurch gekennzeichnet, dass** die Indikatorwand (1020) ein die Aussparung (1024) der Schnappscheibe (1022) bedeckendes Ventilsystem aufweist, vorzugsweise in Form eines Folienventils (1026), das dazu dient, Gase aus der Kammer (1005) abzusaugen und dem Eindringen von Gasen in die Kammer (1005) entgegenzuwirken.

31. Wundversorgungsanordnung nach einem der Ansprüche 21 bis 30, **dadurch gekennzeichnet, dass** ein in die Kammer (1005) mündender Kanal (1050) durch den Kammermantel (1010) verläuft.

32. Wundversorgungsanordnung nach Anspruch 27, **dadurch gekennzeichnet, dass** eine umlaufende Außennut (1012) zur Aufnahme eines Schlauches (1200), der die Kammer (1005) mit dem Wundraum verbindet und vorzugsweise in der Außennut (1012) aufgewickelt ist, in der von der Kammer (1005) abgewandten Begrenzungsfläche des Kammermantels (1010) vorgesehen ist, wobei der Kanal (1050) auf seiner von der Kammer (1005) abgewandten Seite in die äußere Nut (1012) mündet.

33. Wundversorgungsanordnung nach einem der Ansprüche 21 bis 32, **dadurch gekennzeichnet, dass** die Nut (1012) Verriegelungsmittel (1013) zum Verriegeln des aus der Nut (1012) austretenden Schlauchs (1200) aufweist.

34. Wundversorgungsanordnung nach Anspruch 32 oder 33, **dadurch gekennzeichnet, dass** eine Aussparung durch mindestens eine Wand der Außennut (1012) verläuft, um Flüssigkeit aus dieser Aussparung abzulassen.

35. Wundversorgungsanordnung nach den Ansprüchen 21 bis 34, **dadurch gekennzeichnet, dass** die Kammer (1005) durch eine zumindest teilweise transparente Überwachungswand (1030) auf der von der Indikatorwand (1020) abgewandten Seite begrenzt ist.

36. Wundversorgungsanordnung nach Anspruch 35, **dadurch gekennzeichnet, dass** ein mit der Indikatorwand (1020) gekoppeltes Indikatorelement (1040) in der Kammer (1005) angeordnet ist, wobei das Indikatorelement (1040) beim Erreichen der Endposition in Richtung der Überwachungswand (1030) bewegt wird und vorzugsweise mit der Überwachungswand (1030) in Kontakt kommt.

37. Wundversorgungsanordnung nach Anspruch 35 oder 36, **dadurch gekennzeichnet, dass** die Überwachungswand (1030) verformbar ist und mit der der Kammer (1005) zugewandten Begrenzungsfläche der Indikatorwand (1020) in Kontakt kommt, wenn ein vorbestimmter Unterdruck auf die Kammer (1005) aufgebracht wird.

38. Wundversorgungsanordnung nach einem der Ansprüche 7 bis 37, **dadurch gekennzeichnet, dass** die Indikatorvorrichtung einen fluidisch mit dem Wundraum verbundenen Schlauch (700) aufweist, wobei der Schlauch (700) einen dem Wundraum zugewandten unterdruckstabilen Abschnitt (701) und einen dem Wundraum abgewandten kompressiblen Abschnitt (702), der an seinem vom Wundraum abgewandten Ende flüssigkeitsdicht verschlossen ist, aufweist, wobei der Schlauch (700) teilweise mit einer Flüssigkeit (703), insbesondere Weißöl, gefüllt ist.

39. Wundversorgungsanordnung nach einem der vorhergehenden Ansprüche, **gekennzeichnet durch** eine abnehmbar mit dem Ventilsystem (1000) koppelbare Saugvorrichtung (2000).

40. Wundversorgungsanordnung nach Anspruch 39, **dadurch gekennzeichnet, dass** die Saugvorrichtung einen Dichtungsflansch umfasst, der um mindestens ein Einwegventil des Ventilkörpers (1010, 1020, 1030) und/oder der Indikatorvorrichtung herum verläuft, um eine Dichtungsverbindung zwischen der Ventilkammer (1005) und/oder einer Kammer (1005) der Indikatorvorrichtung (1000) und der Saugkammer (2011) herzustellen, die das Austreten von Gasen aus der Ventilkammer (1005) und/oder einer Kammer der Anzeigevorrichtung in mindestens eine Saugkammer (2011) der Saugvorrichtung (2000) ermöglicht, wobei die Saugvorrichtung (2000) vorzugsweise eine Aufnahme für den Ventilkörper aufweist.

41. Wundversorgungsanordnung nach Anspruch 39 oder 40, **dadurch gekennzeichnet, dass** die Saugvorrichtung (2000) betätigt werden kann, um Gase aus der vorzugsweise in der Aufnahme gehaltenen Ventilkammer des Ventilkörpers abzusaugen.

42. Wundversorgungsanordnung nach einem der Ansprüche 39 bis 41, **gekennzeichnet durch** mindestens einen Saugkolben (2010), der vorzugsweise gegen die Vorspannkraft einer Vorspannvorrichtung (2012) in die Saugkammer (2011) eingeführt werden kann.

43. Wundversorgungsanordnung nach Anspruch 42, **dadurch gekennzeichnet, dass** mindestens ein weiteres Ventilsystem (2013) dem mindestens einen Saugkolben (2010) zugehörig ist, wobei das Ventilsystem (2013) die Abgabe von Gasen aus der Saugkammer (2011) in die Umgebung ermöglicht, wenn der Saugkolben (2010) in die Saugkammer (2011) eingeführt ist und dem Eindringen von Umgebungsluft in die Ventilkammer (1005) entgegenwirkt, wenn der Saugkolben (2010) aus der Saugkammer (2011) entfernt wird.

44. Wundversorgungsanordnung nach einem der vorhergehenden Ansprüche, **gekennzeichnet durch** ein ein vorzugsweise absorbierendes Material wie beispielsweise einen Superabsorber umfassendes, in den Wundraum einführbares Wundfüllmaterial (120).

45. Wundversorgungsanordnung nach einem der Ansprüche 39 bis 44, **dadurch gekennzeichnet, dass** die Absaugvorrichtung eine Pumpe aufweist, insbesondere eine elektromechanische Pumpe.

## Revendications

1. Système de soin de plaie pour thérapie à pression réduite comprenant un dispositif de couverture (100) doté, au moins par endroits, d'un agent adhésif conçu pour fixer le dispositif de couverture (100) sur la peau et situé de préférence sur le pourtour de l'espace entourant la plaie, lequel dispositif peut être fixé sur la peau entourant une plaie et permet de produire un espace clos renfermant la plaie, et comprenant un système de valve (200, 1000) associé au dispositif de couverture, ledit système de valve (200, 1000) pouvant être actionné, d'une part, pour aspirer les gaz hors de l'espace entourant la plaie et, d'autre part, pour empêcher la pénétration des gaz dans ledit espace entourant la plaie une fois le dispositif de couverture (100) fixé sur la peau entourant la plaie, le système de valve (200, 1000) pouvant être couplé de manière détachable à un dispositif d'aspiration (300, 1100, 2000) permettant d'aspirer les gaz hors de l'espace entourant la plaie, ledit système de valve (200, 1000) étant couplé au dispositif de couverture (100) au moyen d'une ligne pour fluides, notamment un tube (8, 1200),
**caractérisé en ce que** le système de valve (200, 1000) présente un corps de valve (15, 1010) doté d'une chambre de valve (1005) destinée à recevoir les gaz issus de l'espace entourant la plaie, ledit tube (8, 1200) couplé au dispositif de couverture (100) menant à la chambre de valve (1005), le système de valve (200, 1000) comprenant une valve à feuille (1026) et la chambre de valve (1005) étant au moins partiellement délimitée par un boîtier rigide (1010).

2. Système de soin de plaie selon la revendication 1, **caractérisé en ce que** la valve de non-retour présente au moins une rainure de valve formée dans un élément de délimitation (2) de préférence de type feuille, qui délimite la chambre de valve.

3. Système de soin de plaie selon l'une quelconque des revendications 1 et 2, **caractérisé en ce que** la chambre de valve est approximativement en forme de lentille avant que les gaz s'en soient échappés et est délimitée par deux éléments de délimitation de type feuille (2, 7) reliés entre eux à proximité de leurs bords de manière étanche aux gaz, et au moins un et de préférence les deux éléments de délimitation sont dotés d'au moins une rainure de valve afin de former une valve à feuille.

4. Système de soin de plaie selon la revendication 2 ou 3, **caractérisé en ce qu'**au moins un élément de délimitation (2) présente une couche de renfort (1) dotée d'un évidement à proximité de l'au moins une rainure de valve.

5. Système de soin de plaie selon l'une quelconque des revendications 1 à 4, **caractérisé en ce que** la ligne pour fluides (8) est rattachée à la surface limite du dispositif de couverture dotée de l'agent adhésif.

6. Système de soin de plaie selon l'une quelconque des revendications 1 à 4, **caractérisé en ce que** la ligne pour fluides (8) traverse le dispositif de couverture et/ou mène à une ouverture qui traverse le dispositif d'étanchéité et/ou le dispositif de couverture présente un orifice d'aspiration conçu pour le couplage de la ligne pour fluides.

7. Système de soin de plaie selon l'une quelconque des revendications précédentes, **caractérisé en ce que** un dispositif indicateur (4, 700, 1000), qui peut être exploité pour afficher la condition de pression réduite dans l'espace entourant la plaie, est associé au dispositif de couverture (100).

8. Système de soin de plaie selon la revendication 7, **caractérisé en ce que** le dispositif indicateur est intégré dans le dispositif de couverture (100).

9. Système de soin de plaie selon la revendication 7, **caractérisé en ce que** le dispositif indicateur (4, 700, 1000) est couplé au moyen d'une ligne pour fluides (8, 1200) au dispositif de couverture (100).

10. Système de soin de plaie selon l'une quelconque des revendications 7 à 9, **caractérisé en ce que** le dispositif indicateur (4, 1000) est intégré dans le système de valve (200, 1000), notamment dans la chambre de valve (1005) du système de valve (200, 1000).

11. Système de soin de plaie selon l'une quelconque des revendications 7 à 10, **caractérisé en ce que** le dispositif indicateur présente deux couches indicatrices (4, 6) espacées l'une de l'autre et approximativement parallèles l'une à l'autre, lesquelles peuvent être comprimées, au moins dans certaines régions, contre une force de prétension de manière à réduire l'espacement.

12. Système de soin de plaie selon la revendication 11, **caractérisé par** au moins une couche d'espacement (5) notamment déformable élastiquement, disposée entre les couches indicatrices (4, 6) et pouvant être comprimée en surpassant une force de prétension prédéterminée, celle-ci présentant de préférence au moins une fenêtre.

13. Système de soin de plaie selon l'une quelconque des revendications 9 à 12, **caractérisé en ce que** la ligne pour fluides (8) mène à un espace indicateur formé entre les couches indicatrices (4, 6) et traverse de préférence au moins partiellement la couche d'espacement (5).

14. Système de soin de plaie selon l'une quelconque des revendications 11 à 13, **caractérisé en ce qu'**une surface limite d'une couche indicatrice (6), tournée vers l'autre couche indicatrice (4), présente un profilage et/ou une représentation graphique visibles de l'extérieur lorsque la distance entre les couches indicatrices est réduite.

15. Système de soin de plaie selon l'une quelconque des revendications 7 à 14, **caractérisé en ce que** le dispositif indicateur (1000) présente un boîtier au moins partiellement rigide (500, 1010) qui délimite au moins partiellement une chambre indicatrice (510, 1005) destinée à recevoir les gaz issus de l'espace entourant la plaie.

16. Système de soin de plaie selon la revendication 15, **caractérisé en ce que** deux chambres (510, 520) séparées l'une de l'autre par un dispositif de séparation (600) étanche aux gaz et mobile, notamment déformable, sont formées dans la chambre indicatrice, une première chambre (510) parmi celles-ci pouvant de préférence être reliée à l'espace entourant la plaie au moyen d'une ligne pour fluides, et une deuxième chambre (520) parmi celles-ci pouvant de préférence être reliée à la zone environnante au moyen d'un évidement (622) réalisé dans le boîtier (500).

17. Système de soin de plaie selon la revendication 16, **caractérisé par** un dispositif de prétension (640) logeant de préférence dans la première chambre (510), et comprenant notamment un ressort de compression au moyen duquel le dispositif de séparation (600), réalisé de préférence sous la forme d'une membrane déformable, est maintenu dans une position initiale et contre la force de prétension duquel le dispositif de séparation (600) est déplacé vers une position terminale lorsqu'une pression réduite est appliquée à la première chambre (510).

18. Système de soin de plaie selon la revendication 17, **caractérisé en ce que** le dispositif de séparation (600) vient au moins partiellement en contact avec une surface limite de préférence plane du boîtier (500) de préférence cylindrique, notamment cylindrique circulaire, lorsqu'il parvient en position terminale.

19. Système de soin de plaie selon l'une ou l'autre des revendications 16 et 17, **caractérisé en ce que** sont disposées, dans l'une des chambres, de préférence dans la première chambre (510), deux douilles (620, 630) qui s'emboîtent l'une dans l'autre et qui peuvent se déplacer l'une par rapport à l'autre contre la force de prétension du dispositif de prétension (640), l'une desdites douilles (620, 630) étant couplée à une paroi de boîtier (540) et l'autre étant couplée au dispositif de séparation (600).

20. Système de soin de plaie selon l'une quelconque des revendications 16 à 18, **caractérisé en ce qu'**une paroi de boîtier délimitant la première chambre du boîtier est dotée d'une valve antiretour, notamment une valve à feuille, au moyen de laquelle une pression réduite peut être appliquée à la première chambre et à l'espace entourant la plaie.

21. Système de soin de plaie selon l'une quelconque des revendications précédentes, **caractérisé par** une chambre (1005) en communication fluidique avec l'espace entourant la plaie, de préférence une chambre (1005) sensiblement cylindrique, par exemple la chambre de valve et/ou la chambre indicatrice, qui est délimitée par une enveloppe de chambre circonférentielle (1010) et présente une paroi indicatrice (1020) qui forme de préférence un côté avant de la chambre (1005) et est mobile entre une position initiale et une position finale, la paroi indicatrice (1020) pouvant passer de la position initiale à la position finale sous l'effet de l'application de pression réduite à la chambre (1005).

22. Système de soin de plaie selon l'une quelconque des revendications précédentes, **caractérisé en ce que**, dans la position initiale, la paroi indicatrice (1020) est incurvée de manière convexe vers l'extérieur de l'espace qui est extérieur à la chambre (1005).

23. Système de soin de plaie selon la revendication 21 ou 22, **caractérisé en ce que** la paroi indicatrice (1020) est incurvée vers la chambre (1005) dans la position finale.

24. Système de soin de plaie selon l'une quelconque des revendications 21 à 23, **caractérisé en ce que** la paroi indicatrice (1020) présente un disque à déclic (1022) incurvé vers l'extérieur à l'état non sollicité.

25. Système de soin de plaie selon la revendication 24, **caractérisé en ce que** le disque à déclic (1022) est traversé par un évidement (1024) disposé de préférence dans la région de crête de la courbure et destiné à appliquer une pression réduite à la chambre (1005) et à l'espace entourant la plaie.

26. Système de soin de plaie selon l'une quelconque des revendications 21 à 25, **caractérisé en ce qu'**une zone de bord circonférentielle de la paroi indicatrice (1020) loge dans une rainure intérieure (1014) de l'enveloppe de chambre (1010) qui est ouverte vers la chambre (1005).

27. Système de soin de plaie selon la revendication 26, **caractérisé en ce que**, dans la position initiale et/ou la position finale de la paroi indicatrice (1020), un espace est formé entre le fond de la rainure intérieure et la zone de bord de la paroi indicatrice (1020).

28. Système de soin de plaie selon l'une ou l'autre des revendications 26 et 27, **caractérisé en ce que** des zones des parois de la rainure intérieure venant en contact avec la zone de bord circonférentielle de la paroi indicatrice (1020) sont formées au moins partiellement dans un matériau flexible (1016).

29. Système de soin de plaie selon l'une quelconque des revendications 21 à 28, **caractérisé en ce qu'**une collerette (1060) circonférentielle pour le couplage par complémentarité de forme d'une tête d'aspiration d'un dispositif d'aspiration (1100) est formée dans la région de jonction entre la paroi indicatrice (1020) et l'enveloppe de chambre (1010), ladite collerette (1060) formant de préférence une butée limitant l'avancée de la tête d'aspiration vers l'évidement (1024) traversant le disque à déclic (1022).

30. Système de soin de plaie selon l'une quelconque des revendications 25 à 29, **caractérisé en ce que** la paroi indicatrice (1020) présente un système de valve recouvrant l'évidement (1024) du disque à déclic (1022), de préférence sous la forme d'une valve à feuille (1026), destiné à aspirer les gaz hors de la chambre (1005) et empêchant la pénétration des gaz dans la chambre (1005).

31. Système de soin de plaie selon l'une quelconque des revendications 21 à 30, **caractérisé en ce qu'**un canal (1050) menant à la chambre (1005) traverse l'enveloppe de chambre (1010).

32. Système de soin de plaie selon la revendication 27, **caractérisé en ce qu'**une rainure extérieure (1012) circonférentielle destinée à loger un tube (1200), qui relie la chambre (1005) à l'espace entourant la plaie et qui est de préférence enroulé dans la rainure extérieure (1012), est prévue dans la surface limite de l'enveloppe de chambre (1010) détournée de la chambre (1005), la canal (1050) menant à la rainure extérieure (1012) sur son côté détourné de la chambre (1005).

33. Système de soin de plaie selon l'une quelconque des revendications 21 à 32, **caractérisé en ce que** la rainure (1012) présente des moyens de verrouillage (1013) permettant de verrouiller le tube (1200) sortant de la rainure (1012).

34. Système de soin de plaie selon la revendication 32 ou 33, **caractérisé en ce qu'**un évidement traverse au moins une paroi de la rainure extérieure (1012) afin d'évacuer du liquide dudit évidement.

35. Système de soin de plaie selon les revendications 21 à 34, **caractérisé en ce que** la chambre (1005) est délimitée par une paroi de surveillance (1030) au moins partiellement transparente sur le côté détourné de la paroi indicatrice (1020).

36. Système de soin de plaie selon la revendication 35, **caractérisé en ce qu'**un élément indicateur (1040) couplé à la paroi indicatrice (1020) est disposé dans la chambre (1005), lequel élément indicateur (1040) se rapproche de la paroi de surveillance (1030) en atteignant la position finale et vient de préférence en contact avec la paroi de surveillance (1030).

37. Système de soin de plaie selon la revendication 35 ou 36, **caractérisé en ce que** la paroi de surveillance (1030) est déformable et vient en contact avec la surface limite de la paroi indicatrice (1020) tournée vers la chambre (1005) lorsqu'une pression réduite prédéterminée est appliquée à la chambre (1005).

38. Système de soin de plaie selon l'une quelconque des revendications 7 à 37, **caractérisé en ce que** le dispositif indicateur présente un tube (700) en communication fluidique avec l'espace entourant la plaie, lequel tube (700) présente une section stable à la pression réduite (701) tournée vers l'espace entourant la plaie et une section compressible (702) qui est détournée de l'espace entourant la plaie et qui est fermée de manière étanche aux fluides à son extrémité détournée de l'espace entourant la plaie, le tube (700) étant rempli partiellement d'un liquide (703), notamment d'huile blanche.

39. Système de soin de plaie selon l'une quelconque des revendications précédentes, **caractérisé par** un dispositif d'aspiration (2000) pouvant être couplé de manière détachable au système de valve (1000).

40. Système de soin de plaie selon la revendication 39, **caractérisé en ce que** le dispositif d'aspiration comprend une bride d'étanchéité entourant au moins une valve antiretour du corps de valve (1010, 1020, 1030) et/ou du dispositif indicateur afin d'établir une communication étanche entre la chambre de valve (1005) et/ou une chambre (1005) du dispositif indicateur (1000) et la chambre d'aspiration (2011), permettant aux gaz de passer de la chambre de valve (1005) et/ou d'une chambre du dispositif indicateur vers au moins une chambre d'aspiration (2011) du dispositif d'aspiration (2000), le dispositif d'aspiration (2000) présentant de préférence un récepteur destiné au corps de valve.

41. Système de soin de plaie selon la revendication 39 ou 40, **caractérisé en ce que** le dispositif d'aspiration (2000) peut être actionné pour aspirer les gaz hors de la chambre de valve du corps de valve qui est de préférence retenu dans le récepteur.

42. Système de soin de plaie selon l'une quelconque des revendications 39 à 41, **caractérisé par** au moins un piston d'aspiration (2010) pouvant de préférence être introduit dans la chambre d'aspiration (2011) contre la force de prétension d'un dispositif de prétension (2012).

43. Système de soin de plaie selon la revendication 42, **caractérisé en ce qu'**au moins un autre système de valve (2013) est associé à l'au moins un piston d'aspiration (2010), lequel système de valve (2013) permet à des gaz d'être évacués de la chambre d'aspiration (2011) vers la zone environnante lorsque le piston d'aspiration (2010) est introduit dans la chambre d'aspiration (2011) et empêche la pénétration de l'air ambiant dans la chambre de valve (1005) lorsque le piston d'aspiration (2010) est retiré de la chambre d'aspiration (2011).

44. Système de soin de plaie selon l'une quelconque des revendications précédentes, **caractérisé par** une matière obturatrice de plaie (120), comprenant de préférence une matière absorbante, par exemple une matière superabsorbante, susceptible d'être introduite dans l'espace entourant la plaie.

45. Système de soin de plaie selon l'une quelconque des revendications 39 à 44, **caractérisé en ce que** le dispositif d'aspiration présente une pompe, notamment une pompe électromécanique.
